# EUROPEAN PATENT SPECIFICATION

(11) **EP 1 321 035 B1**
(45) Date of publication and mention of the grant of the patent: **10.05.2006**
(21) Application number: 02022525.6
(22) Date of filing: 07.10.2002
(51) Int. Cl.: A01M 1/20, A61L 9/03

(54) **Heater for a diffusing device**
Heizkörper für einen Diffusor
Elément de chauffage pour un diffuseur

(30) Priority: 20.12.2001 IT MI20012722
(43) Date of publication of application: 25.06.2003
(73) Proprietor: ZOBELE HOLDING S.P.A., 38100 Trento (IT)
(72) Inventor: Zobele, Franco, 38100 Trento (IT)
(74) Representative: Petruzziello, Aldo

(56) References cited:
- EP-A- 0 689 766
- EP-A- 0 998 947
- DE-U- 9 102 695
- GB-A- 2 357 035
- US-A1- 2003 071 030

## Description

The present invention concerns a heater for a diffusing device for diffusing solutions containing active ingredients, such as deodorants, insecticides, disinfectants or similar substances.

A diffusing device generally comprises a heater which may be coupled to a respective refill provided with an evaporating wick for diffusing solutions.

At present various types of diffusing devices are available on the market comprising an electric heater provided with an electric plug for connection to an electric socket so as to feed an electric resistance which heats up. The heater is coupled to a refill containing a solution and provided with an evaporating wick which, when heated by contact with the resistance, diffuses the solution.

EP-A-0 689 766 discloses an apparatus for vaporising insecticide solutions comprising a bottom and a top half-shell designed to receive a portion of a wick and a further functional unit positioned between the bottom and the top half-shells and providing means for heating the wick to cause the evaporation of a insecticide solution.

EP-A-0 998 947 discloses a heater comprising a single electrical heating means, which is positioned on one side of the hole provided for the wick and is supported and power supplied by electrodes having a high heat conducing coefficient and fully surrounding said hole to heat the wick uniformly.

The heaters of the diffusing devices according to the prior art have some drawbacks.

These heaters are rather complex, bulky and expensive, especially on account of their internal structure and of the respective coupling with the electric heating resistance.

Another inconvenient aspect of some known heaters lies in the fact that the refill is solidly fixed to the heater. Consequently, depending on the orientation (vertical, horizontal or oblique) of the electric socket that receives the plug of the heater, it may occur that the refill is incorrectly oriented, causing leakage of the solution that it contains.

The object of the present invention is to eliminate the drawbacks of the prior art, supplying a heater for a diffusing device which is economic and simple to make.

Another object of the present invention is to provide such a heater for a diffusing device which has a limited number of parts, having a simplified structure, easily assembled together.

Another object of the present invention is to provide such a heater which is not bulky, is practical, extremely versatile and suitable to be applied on various types of diffusing devices.

Another object of the present invention is to provide such a heater for a diffusing device which is suitable to be applied on an electric socket, having any orientation.

These objects are achieved according to the invention with the characteristics listed in the enclosed independent claim 1.

Advantageous embodiments of the invention are given in the dependent claims.

The heater for a diffusing device according to the invention comprises a bottom half-shell which may be coupled with a container containing a solution to be diffused and a top half-shell which may be coupled with the bottom half-shell and provided with an opening designed to receive a portion of a wick which is in contact with the solution held in the container.

In the heater there are resistive means which increase the temperature, when current passes through them, to heat the wick so as to cause the evaporation of the solution from the wick. These resistive means are fed by electric supply means connected to the heater.

In the heater there is a tang, substantially cylindrical in shape, which comprises an axial hole to allow the passage of the wick and a substantially annular seat for holding the resistive means in order to heat the wick.

This structure of the heater is extremely simple and the heater may be advantageously obtained by two half-shells made by moulding heat-resistant plastic materials.

Further characteristics of the invention will appear more clearly from the following detailed description which refers to embodiments taken purely as examples and therefore without limitation, illustrated in the enclosed drawings, in which:
Fig. 1 is a perspective view of a diffusing device with a heater made of two half-shells, according to a first embodiment of the invention;
Fig. 2 is a plain view of the top half-shell of the heater in Fig. 1, taken from the part facing inward;
Fig. 3 is an axial sectional view according to the sectional plane III-III in Fig. 2;
Fig. 4 is an axial sectional view according to the sectional plane IV-IV in Fig. 2;
Fig. 5 is an top plain view of the electric plug of the heater according to the invention;
Fig. 6 is a plain view of the bottom half-shell of the heater in Fig. 1, taken from the part facing inward;
Fig. 7 is an axial sectional view according to the sectional plane VII-VII in Fig. 6;
Fig. 8 is a plain view of the top half-shell seen from the internal part with an assembled electric plug;
Fig. 9 is a top plain view of a support element of the electric resistances but not according to the invention;
Fig. 10 is an axial sectional view according to the sectional plane X-X in Fig. 9;
Fig. 11 is a cross sectional view according to the sectional plane XI-XI in Fig. 10;
Fig. 12 is a top plain view of a support element of the electric resistances assembled on the electric plug;
Fig. 13 is an exploded view of a heater according to a second embodiment of the invention, in which the top half-shell and the bottom half-shell are seen in plain from the part facing inward and the electric plug is seen from the rear.

With the aid of the Figures the heater according to the invention is described.

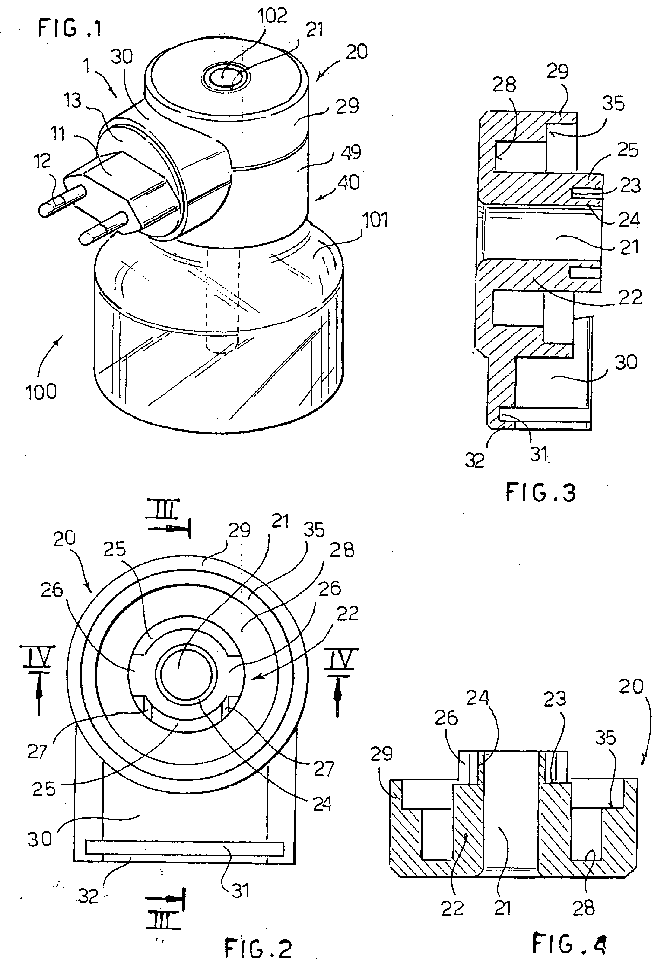

With reference to Fig. 1 a diffusing device is illustrated, indicated overall with the reference number 100. The diffusing device 100 comprises a refill 101, a wick 102 and a heater indicated overall with the reference number 1.

The refill 101 is in the form of a container or bottle containing a solution comprising active ingredients. The heater 1 is applied to the container 101 and supports the wick 102. The wick 102 is a porous element of a substantially cylindrical shape having one end immersed in the solution inside the refill 101 and the other end protruding from the heater 1 to diffuse the solution in the outside environment.

The heater 1 comprises an electric plug 10 suitable to be inserted in an electric power socket to feed the resistive elements which heat up when current passes through them. These resistive elements, located inside the heater 1, cause the wick to be heated by conduction or convection. When heated, the wick 102, impregnated with solution, allows the evaporation of the solution in the outside environment.

The heater 1 according to the invention comprises a top half-shell 20 having at least one opening communicating with the free end of the wick 102 and a bottom half-shell 40 suitable to be applied to the mouth of the container 101.

As shown also in Figs. 2 - 4, the top half-shell 20 has a body of a substantially cylindrical shape defined by a cylindrical external part 29. Projecting radially from the external wall 29 is a connector 30 with a hollow semi-cylindrical shape for receiving the electric plug 10. At the end of the connector 30 is a semi-annular seat 31 defined by a protruding profile 32 in the shape of a semi-circle.

The body of the half-shell 20 axially has a cylindrical tang 22 having an axial through hole 21 suitable to allow the insertion of the wick 102.

On the cylindrical tang 22 there is an annular seat 23 bounded by a cylindrical internal wall 24 around the axial hole 21 and a cylindrical external wall 25. The cylindrical external wall 25 has two slots 26, placed in diametrically opposite positions, at an angle of about 90° with respect to the axis of the semi-cylindrical connector 30. The slots 26 separate the cylindrical external wall 25 of the tang 22 into two wall sections. In the wall section 25 nearer the connector 30 there are two cuts 27 spaced apart with each other.

To lighten the top half-shell 20, between the external surface of the cylindrical tang 22 and the external wall 29 of the body is provided a circular cavity 28.

The external wall 29 of the body of the top half-shell 20 has at the bottom edge a annular abutting surface 35 which protrudes radially inward, so as to form a step.

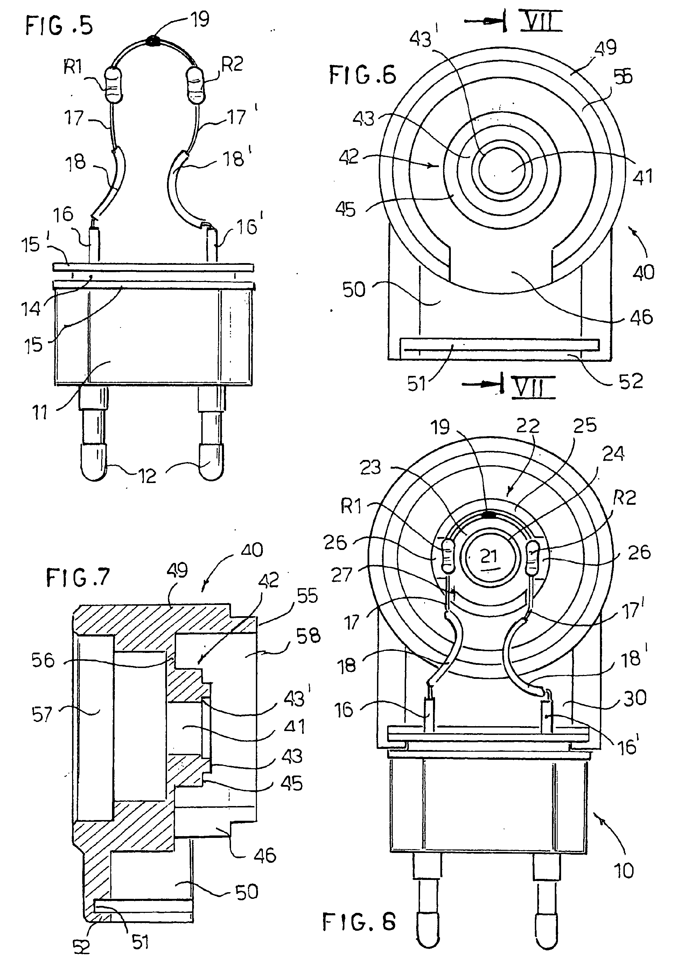

As shown in Fig. 5, the plug 10 comprises a substantially rectangular body 11 from which two pins 12, suitable to be inserted in the respective holes of an electric power socket, protrude at the front.

The body 11 of the plug is mounted on a flange 13 having the shape of a cylindrical plate. Around the circumference edge of the flange 13 is a annular seat 14 defined between two circular projections 15, 15'. In this way the projection 15' of the flange 13 engages in the seat 31 of the connector 30 and the projection 32 of the connector 30 engages in the seat 14 of the flange 13.

Protruding from the rear of the flange 13 are two electric contacts 16, 16' electrically connected to the respective poles 12. Fixed to the electric contacts 16, 16', for example by crimping or welding, are two respective electric cables 17, 17' bearing respective electric resistances R1 and R2. The electric cables 17, 17' are covered by respective insulating sheaths 18, 18' and are connected at their ends by welding 19. On the resistances R1 and R2 there may be a heat-resistant sheath (not shown in Fig. 5).

In this way the two resistances R1 and R2 are connected in series. So when the plug 10 is inserted in a power socket, an electric current passes through the resistances R1 and R2 which increase in temperature.

As shown in Fig. 8, the plug 10 is fitted on the top half-shell 20. The projection 15 of the flange 13 of the plug engages in the seat 31 of the connector 30 and the projection 32 of the connector 30 engages in the seat 14 of the flange 13 of the plug 10.

The resistances R1 and R2 are positioned in the seat 23 of the tang 22 exactly corresponding to the slots 26 in the external wall 25 of the tang. The electric cables 17, 17' pass through the cuts 27 in the external wall 25 of the tang and the electric contacts 16, 16' are contained inside the connector 30.

It should be noted that the resistances R1 and R2 are in contact with the internal wall 24 which bounds the hole 21 through which the wick passes. Consequently the resistances R1 and R2 exchange heat with the wall 24 which in turn gives heat to the wick 102 which heats up to an optimum temperature (about 60°C) to allow the diffusion of the solution.

The function of the slots 26 is to avoid heat loss from the resistances R1 and R2 towards the outside. The function of the cuts 27 is to hold the electric cables 17, 17' to block the resistances R1 and R2 in position.

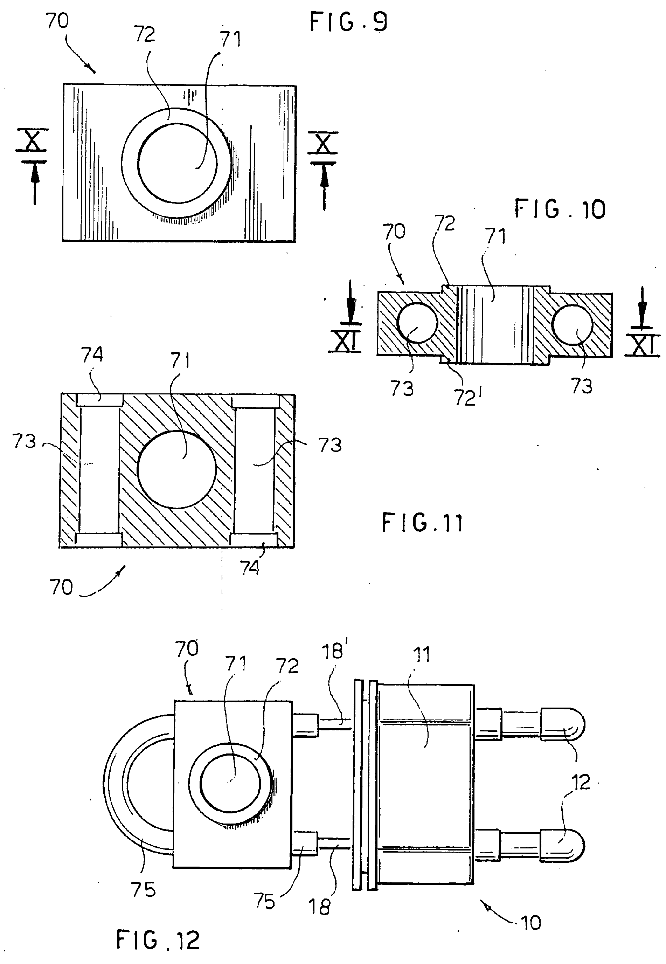

As shown also in Figs. 6 and 7, the bottom half-shell 40 has a body of a substantially cylindrical shape defined by a cylindrical external wall 49. Projecting radially from the external wall 49 is a connector 50 with a hollow semi-cylindrical shape, substantially the same as the connector 30 of the top half-shell. At the end of the connector 50 is a semi-annular seat 51 defined by a protruding end part 52.

The bottom half-shell 40 has a transverse partition 56 placed in a median position to divide a bottom chamber 57 suitable to hold the neck of the container 101 and a top chamber 58 for engaging with the top half-shell 20.

On the transverse partition 56 is an axial tang 42 axially presenting a through hole 41 suitable to allow the insertion of the wick 1. The tang 42 has in the top chamber 58 an external annular abutting surface 45 and a central annular abutting surface 43, which bounds an internal shoulder 43'.

The external wall 49 of the bottom half-shell has a slot 46 in the direction of the connector 50 to allow the passage of the sheaths 18, 18' of the electric cables of the plug 10.

The external cylindrical wall 49 of the bottom half-shell 40 has on its upper edge a annular projection 55 so as to form a step. After the plug 10 has been fitted in the top half-shell 20, as shown in Fig. 8, the bottom half-shell 40 is coupled with the top half-shell 20. Exactly the central abutting surface 43 of the tang 42 of the bottom half-shell abuts against the resistances R1 and R2 in the seat 23 of the tang of the top half-shell, blocking the resistances R1 and R2 in position.

The internal cylindrical wall 24 of the tang of the top half-shell 20 goes into the axial hole 41 in the bottom half-shell 40 guaranteeing the centring of the two half-shells, abutting against the shoulder 43'.

The external abutting surface 45 of the tang of the bottom half-shell abuts against the external wall of the tang of the top half-shell and the annular projection 55 of the external wall 49 of the bottom half-shell abuts against the abutting surface 35 of the internal wall of the top half-shell.

Moreover the sheaths 18, 18' of the electric cables of the plug 10 pass through the slot 46 in the external wall of the bottom half-shell and the projection 15 of the flange 13 of the plug engages in the seat 51 of the connector 50 of the bottom half-shell and the projection 52 of the connector 50 of the bottom half-shell engages in the seat 14 of the flange 13 of the plug 10.

Based on this the plug 10 is rotatably supported by two connectors 30 and 50 respectively of the top and of the bottom half-shell. So the plug 10 can be oriented to suit the position of the electric socket in which it is to be inserted. In this way the bottom half-shell 40 with the respective bottle 101 is always facing downwards to avoid leakage of the solution contained in the bottle 101.

The bottom half-shell 40 and the top half-shell 20 may be obtained in a single piece by moulding plastic materials. Any plastic material that has good heat-resisting characteristics may be used for the purpose. Preferably PP (Polypropylene) may be used, which resists a temperature of more than 120 °C.

For high working temperatures, plastics with better heat-resisting characteristics may be used, such as PBT (Poly-Butylene-Terephthalate) or PPS (Poly-Phenyl-Sulphide). Alternatively, when insecticide solutions have to be evaporated at a higher evaporation point (temperature) (for example up to 120°C), the seat 23 of the tang 21 of the top half-shell wherein are housed the resistances R1 and R2 and the abutting part 43 of the tang 41 of the bottom half-shell which is to abut against the resistances R1 and R2 may be made of plastic material having high resistance to heat and the other parts of the half-shells 40 and 20 far from the resistances R1 and R2 can be made of more economic plastic material, having a lower resistance to heat.

Figs. 9 - 12 show a variation of the first embodiment of the invention, in which the seat that houses the resistances R1 and R2 is made differently. In this variation, which does not form part of the invention, there is an element 70 for housing the resistances. The housing element 70 has the shape of a substantially parallelepiped block presenting through hole 71 in a central position having a sufficient diameter to allow the passage of the wick 102.

On the top and bottom surface of the housing element 70 around the axial hole 71 there are two annular projections 72, 72' suitable to engage in respective shoulders provided around the hole 21 in the tang 22 of the top half-shell 20 and around the hole 41 in the tang 42 of the bottom half-shell 40. In this case clearly the tang 22 of the top half-shell 20 will not have the walls 26 and 26 which define the seat 23.

The housing element 70 has two housings 73 in the form of transverse through channels, arranging parallel to each other, in diametrically opposite positions with respect to the axial hole 71. The housings 73 have a sufficient diameter to allow them to house the resistances R1 and R2. The housings 73 end in openings with a larger diameter 74 to facilitate the insertion of the resistances R1 and R2.

The resistances R1 and R2 are inserted in the respective housings 73, and are then connected to each other in series by welding their end terminals. As shown in Fig. 12, an insulating sheath 75 is used, in the form of a heat-resistant tubular cord, to cover the resistances R1 and R2. The purpose of the sheath 75 is to provide further protection of the electric cables 17 and of the resistances R1 and R2 in the event of the heater 1 being deteriorated as a result of abnormal heating conditions which may arise, for example, if the plug of a heater design to operate at a mains voltage of 110V is inserted in a socket with mains voltage 220V.

The bottom half-shell 40 and the top half-shell 20 may be coupled together by welding, for example heat-sealing, ultrasound welding and similar. Alternatively the bottom half-shell 40 and the top half-shell 20 may be coupled together by gluing or with suitable fixing means, such as screws, hooks, joints and similar. For example, on the top half-shell 20 and on the bottom half-shell 40 respective top and bottom hooks may be fitted with couple together. These top and bottom hooks may be an integral part of the respective half-shells.

The bottom half-shell 40 and the top half-shell 20 may also be made up of several separate parts which are then assembled together.

The plug 10 may also have a different number of electric resistances, for example only one resistance or more than two resistances always connected together in series.

The heater 1 may contain inside it regulating devices for controlling the quantity of solution that evaporates in the air in the unit of time. These regulating devices, already known, are based on the moving of the container 101 and/or of the wick 102.

In the present description the tang 22 with the seat 23 for holding the resistances R1 and R2 has been provided in the top half-shell 20 and the tang 42 with the abutting surface 43 for abutting against the resistances R1 and R2 has been provided in the bottom half-shell 40. However, in an obvious manner for a skilled in the art, the tang 22 that holds the resistances can be provided in the bottom half-shell 40 and the contrasting tang 42 can be provided in the top half-shell 20.

Moreover, in the present description an electric plug 10 has been illustrated which is designed to be inserted in an electric power socket for feeding the resistances R1 and R2. However, instead of the electric plug 10, other supply means may be provided, such as batteries, coupled to the heater 1.

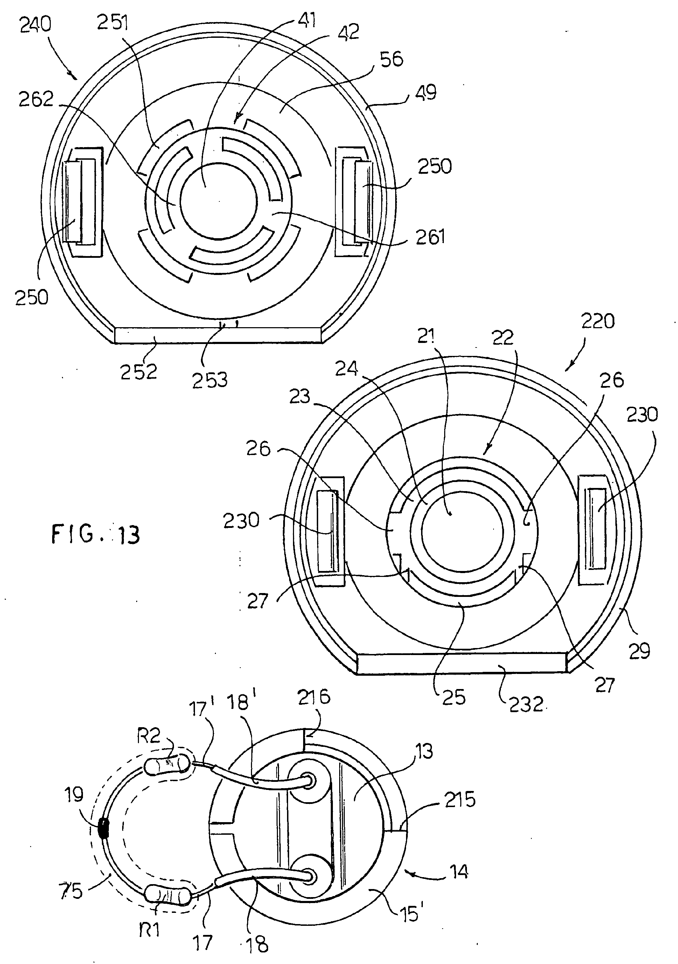

Fig. 13 shows a heater 200 according to a second embodiment of the invention, in which elements the same as or similar to those already illustrated are indicated with the same reference numbers and their detailed description is omitted.

The heater 200 comprises a top half-shell 220, a bottom half-shell 240 and a plug 10. The top half-shell 220 is substantially similar to the top half-shell 20 of the first embodiment, except for the fact that the top half-shell 220 has the shape of a truncated spherical cap and it does not have the connector 30.

Instead, in the edge of its external wall 29, it presents a connecting profile 232 cut in the shape of a semi-circle which has a thickness sufficient to engage in the annular seat 14 of the flange 13 of the plug 10.

Moreover in the external wall 29 of the top half-shell 220 there are two seats or slots 230 located in diametrically opposite positions in the direction of the slots 26 of the tang 22.

The bottom half-shell 240, like the top half-shell 220, has the shape of a truncated spherical cap and presents in the edge of its external wall 49 a profile in the shape of a semi-circle 252 which has a thickness sufficient to engage in the annular seat 14 of the flange 13 of the plug 10. It should be noted that behind the profile 252 there is a pawl 253.

The projection 15' which defines the annular seat 14 of the flange 13 of the plug 10 is located on the flange 13 for an angle of about 270° leaving a part of the circumference of the flange 13 uncovered for an angle of about 90°. In this way the projection 15' has two abutting ends 215 and 216. So, when the flange 13 of the plug 10 is fitted in the connectors 232 and 252 it can rotate with respect to the half-shells for an angle of about 90°. That is, until the abutting surfaces 216 and 215 are abutted against the pawl 253 of the bottom half-shell.

In the side wall 49 of the bottom half-shell 240 there are two flexible fins 250 located in diametrically opposite positions to snap engage into the seats 230 in the side wall 29 of the top half-shell.

In the bulkhead 56, which separates the bottom chamber for holding the bottle 101 from the top chamber for coupling to the top half-shell, there are four air intakes 251. Moreover in the tang 42 of the bottom half-shell there are three slots 260 separates by three bridges 261 which hold a ring 262 that bounds the central hole 41 for the insertion of the wick 102.

In this way, when the two half-shells 20 and 40 are coupled, the bridges 261 abut against the resistances R1 and R2 keeping them in position in the seat 23 of the tang of the top half-shell.

Numerous variations may be made to the present embodiments of the invention and modifications to the details, within the scope of a person skilled in the art, however all falling within the scope of the invention expressed by the enclosed claims.

## Claims

1. Heater (1; 200) for a diffusing device (100) comprising:
- a bottom half-shell (40; 240) which may be coupled with a container (101) containing a solution to be diffused,
- a top half-shell (20; 220) which may be coupled with said bottom half-shell (40; 240) and provided with an opening (21) designed to receive a portion of a wick (102) partially immersed in the solution held in the container (101),
- resistive means (R1, R2) which increase in temperature, when current passes through them, placed in said heater to heat said wick (102) so as to cause the evaporation of said solution, and
- electric supply means (10) coupled to said heater for electrically feeding said resistive means (R1, R2) so as to allow them to increase in temperature,
**characterised in that**
said heater further comprises a substantially cylindrical tang (22), integral to said top half-shell (20; 220) or to said bottom half-shell (40; 240), having an axial through hole (21) to allow the passage of the wick (102) and an annular seat (23) for holding said resistive means (R1, R2), formed within the thickness of said cylindrical tang (22) and defined by a cylindrical internal wall (24) placed around the axial through hole (21) and by a cylindrical external wall (25) placed around said tang, so that said resistive means (R1, R2) can heat the wick (102).

2. Heater according to claim **1**, **characterised in that** said cylindrical external wall (25) which bounds the seat (23) of the resistive means (R1, R2) has openings (26), near the resistive means, suitable to not losing heat towards the outside and to concentrating the heat towards the internal wall (24) which is in contact with the wick (102).

3. Heater according to claim **1**, **characterised in that** in said external wall (25) of the tang (22) wherein is the seat of the resistive means there are two cuts (27) suitable to allow the passage of electric cables (17, 17') connected to the resistive means (R1, R2).

4. Heater according to any one of the previous claims, **characterised in that** said tang (22) supporting the resistive means (R1, R2) is formed axially in said top (20; 220) or respectively in said bottom, (40; 240) half-shell and in said bottom (40; 240) or respectively in said top (20; 220) half-shell there is a corresponding tang (42) presenting an abutting surface (43; 261) suitable to abutting against said resistive means (R1, R2) placed in said seat (23) of the first tang (22) for holding them fixed in position.

5. Heater according to any one of the previous claims, **characterised in that** said resistive means are two electric resistances (R1, R2).

6. Heater according to claim **5**, **characterised in that** said supply means are an electric plug (10) comprising two pins (12) connected by electric cables (17, 17') to said two electric resistances (R1, R2).

7. Heater according to claim **5 or 6**, **characterised in that** said resistances are covered by a heat-resistant insulating sheath (75) acting as protection against possible deterioration of the heater.

8. Heater according to claim **6** or **7**, **characterised in that** said electric plug (10) has a circular flange (13) suitable for rotatable coupling respective connectors (30, 50; 232, 252) of said heater so that it can be turned to suit the orientation of the electric power socket in which it is to be inserted.

9. Heater according to any one of the previous claims, **characterised in that** it comprises regulating devices suitable for moving the container (101) and/or the wick (102) so as to control the quantity of solution diffused in the unit of time.

10. Heater according to any one of the previous claims, **characterised in that** said bottom (40; 240) and top (20; 220) half-shells are formed by injection moulding of plastic materials.

11. Heater according to claim **10**, **characterised in that** said bottom (40; 240) and top (20; 220) half-shells are made of plastic material having adequate heat resistance, such as PP (Polypropylene).

12. Heater according to claim **10**, **characterised in that** at least the parts of said bottom (40; 240) and top (20; 220) half-shells near said resistive elements are made of plastic material having good heat-resisting characteristics, such as PBT (Poly-Butylene-Terephthalate) or PPS (Poly-Phenyl-Sulphide).

13. Heater according to any one of the previous claims, **characterised in that** said bottom (40; 240) and top (20; 220) half-shells are coupled together permanently by welding, such as heat-sealing or ultrasound welding.

14. Heater according to any one of the claims from **1** to **12**, **characterised in that** said bottom (40; 240) and top (20; 220) half-shells are coupled together permanently by fixed or snap couplings.

15. Diffusing device (100), comprising a container (101) containing a solution to be diffused and a wick (102) immersed at least partly in said solution to be diffused contained in the container (101), **characterised in that** it comprises a heater (1; 200) according to any one of the previous claims.

## Patentansprüche

1. Heizvorrichtung (1; 200) für eine Zerstäubungsvorrichtung (100), umfassend:
- eine untere Halbschale (40; 240), welche mit einem Behälter (101) gekoppelt werden kann, der eine Lösung enthält, die zerstäubt werden soll,
- eine obere Halbschale (20; 220), welche mit der unteren Halbschale (40; 240) gekoppelt werden kann und mit einer Öffnung (21) ausgestattet ist, welche so gestaltet ist, um einen Abschnitt eines Dochts (102) aufzunehmen, welcher teilweise in die Lösung, die im Behälter (101) aufgenommen ist, eingetaucht ist,
- Widerstandsmittel (R1, R2), welche ihre Temperatur erhöhen, wenn Strom durch sie hindurchfließt, welche in der Heizvorrichtung angeordnet sind, um den Docht (102) zu erwärmen, um so die Verdunstung der Lösung zu verursachen, und
- elektrische Versorgungsmittel (10), welche mit der Heizvorrichtung zum elektrischen Versorgen der Widerstandsmittel (R1, R2) gekoppelt sind, um sie so ihre Temperatur erhöhen zu lassen,
**dadurch gekennzeichnet, dass**
die Heizvorrichtung des Weiteren einen im Wesentlichen zylindrischen Aufnahmekörper (22) umfasst, welcher einstückig mit der oberen Halbschale (20; 220) oder mit der unteren Halbschale (40; 240) ausgebildet ist, welche ein axiales Durchgangsloch (21), um das Hindurchgehen des Dochtes (102) zu gestatten, und eine ringförmige Aufnahme (23) zum Halten der Widerstandsmittel (R1, R2) aufweist, die innerhalb der Wandstärke des zylindrischen Aufnahmekörpers (22) ausgebildet und durch eine zylindrische innere Wand (24), welche um das axiale Durchgangsloch (21) angeordnet ist, und durch eine zylindrische äußere Wand (25), die um den Aufnahmekörper angeordnet ist, definiert ist, so dass die Widerstandsmittel (R1, R2) den Docht (102) erwärmen können.

2. Heizvorrichtung gemäß Anspruch 1, **dadurch gekennzeichnet, dass** die zylindrische äußere Wand (25), welche die Aufnahme (23) der Widerstandsmittel (R1, R2) begrenzt, Öffnungen (26) nahe der Widerstandsmittel aufweist, welche geeignet sind, keine Wärme in Richtung der Außenseite zu verlieren und die Wärme in Richtung der inneren Wand (24) zu konzentrieren, welche sich in Kontakt mit dem Docht (102) befindet.

3. Heizvorrichtung gemäß Anspruch 1, **dadurch gekennzeichnet, dass** in der zylindrischen äußeren Wand (25) des Aufnahmekörpers (22), in welcher sich die Aufnahme der Widerstandsmittel befindet, zwei Schlitze (27) vorhanden sind, welche geeignet sind, den Durchlass von elektrischen Kabeln (17, 17') zu gestatten, die mit den Widerstandsmitteln (R1, R2) verbunden sind.

4. Heizvorrichtung gemäß jedem der vorangehenden Ansprüche, **dadurch gekennzeichnet, dass** der Aufnahmekörper (22), welcher die Widerstandsmittel (R1, R2) trägt, axial jeweils in der oberen (20; 220) oder in der unteren (40; 240) Halbschale ausgebildet ist und es befindet sich jeweils in der unteren (40; 240) oder in der oberen (20; 220) Halbschale ein entsprechender Aufnahmekörper (42), welcher eine anstoßende Fläche (43; 261) darstellt, welche geeignet ist, um gegen die Widerstandsmittel (R1; R2) zu stoßen, die in der Aufnahme (23) des ersten Aufnahmekörpers (22) angeordnet sind, um sie fest in Position zu halten.

5. Heizvorrichtung gemäß jedem der vorangehenden Ansprüche, **dadurch gekennzeichnet, dass** die Widerstandsmittel zwei elektrische Widerstände (R1, R2) sind.

6. Heizvorrichtung gemäß Anspruch 5, **dadurch gekennzeichnet, dass** die Versorgungsmittel ein elektrischer Stecker (10) sind, welcher zwei Kontaktstifte (12) umfasst, die durch die elektrischen Kabeln (17, 17') mit den Widerstandsmitteln (R1, R2) verbunden sind.

7. Heizvorrichtung gemäß Anspruch 5 oder 6, **dadurch gekennzeichnet, dass** die Widerstände durch eine wärmebeständige Isolierhülle (75) bedeckt sind, welche als Schutz gegen mögliche Schäden an der Heizvorrichtung wirken.

8. Heizvorrichtung gemäß Anspruch 6 oder 7, **dadurch gekennzeichnet, dass** der elektrische Stecker (10) einen kreisförmigen Flansch (13) aufweist, welcher geeignet ist, um die jeweiligen Verbinder (30, 50; 232, 252) der Heizvorrichtung so drehbar zu kuppeln, dass er gedreht werden kann, um der Ausrichtung der elektrischen Stromsteckdose, in welche er eingeschoben wird, zu entsprechen.

9. Heizvorrichtung gemäß jedem der vorangehenden Ansprüche, **dadurch gekennzeichnet, dass** er Reguliervorrichtungen umfasst, welche geeignet sind, um den Behälter (101) und/oder den Docht (102) so zu bewegen, um die Menge der Lösung, welche in der Zeiteinheit zerstäubt werden soll, zu regeln.

10. Heizvorrichtung gemäß jedem der vorangehenden Ansprüche, **dadurch gekennzeichnet, dass** die untere (40; 240) und die obere (20; 220) Halbschale durch Spritzguss von Kunststoffmaterialien ausgebildet sind.

11. Heizvorrichtung gemäß Anspruch 10, **dadurch gekennzeichnet, dass** die untere (40; 240) und die obere (20; 220) Halbschale aus Kunststoffinaterial hergestellt sind, welches zufriedenstellende Wärmebeständigkeit aufweist, wie PP (Polypropylen).

12. Heizvorrichtung gemäß Anspruch 10, **dadurch gekennzeichnet, dass** wenigstens Teile der unteren (40; 240) und der oberen (20; 220) Halbschale nahe der Widerstandselemente aus Kunststoffmaterial hergestellt sind, welches gute Wärmebeständigkeitseigenschaften aufweist, wie PBT (Poly-Butylen-Terephtalat) oder PPS (Poly-Phenyl-Sulfid).

13. Heizvorrichtung gemäß jedem der vorangehenden Ansprüche, **dadurch gekennzeichnet, dass** die untere (40; 240) und die obere (20; 220) Halbschale dauerhaft miteinander durch Verschweißen, wie Heißsiegeln oder Ultraschallschweißen, gekoppelt sind.

14. Heizvorrichtung gemäß jedem der Ansprüche 1 bis 12, **dadurch gekennzeichnet, dass** die untere (40; 240) und die obere (20; 220) Halbschale dauerhaft miteinander durch feste oder einschnappende Kupplungselemente gekoppelt sind.

15. Zerstäubungsvorrichtung (100), umfassend einen Behälter (101), welcher eine Lösung, die zerstäubt werden soll, enthält, und einen Docht (102), welcher wenigstens teilweise in der Lösung, die zerstäubt werden soll und im Behälter (101) enthalten ist, **dadurch gekennzeichnet, dass** sie eine Heizvorrichtung (1; 200) gemäß jedem der vorangehenden Ansprüche umfasst.

## Revendications

1. Élément chauffant (1; 200) pour un dispositif diffuseur (100) comprenant :
- une demi-coque inférieure (40; 240) qui peut être couplée à un récipient (101) contenant une solution à diffuser,
- une demi-coque supérieure (20, 220) qui peut être couplée à ladite demi-coque inférieure (40; 240), est prévue avec une ouverture (21) conçue en vue de recevoir une partie d'une mèche (102) partiellement plongée dans la solution contenue dans le récipient (101),
- des moyens résistifs (R1, R2) dont la température augmente, lorsqu'un courant les traverse, placés dans ledit élément chauffant afin de chauffer ladite mèche (102) pour provoquer l'évaporation de ladite solution, et
- des moyens d'alimentation électrique (10) couplés audit élément chauffant pour alimenter électriquement lesdits moyens résistifs (R1, R2) de sorte à permettre une augmentation de leur température,
**caractérisé en ce que**,
ledit élément chauffant comprend en outre un fût sensiblement cylindrique (22), solidaire de ladite demi-coque supérieure (20; 220) ou de ladite demi-coque inférieure (40; 240), ayant un trou traversant axial (21) afin de permettre le passage de la mèche (102) et un siège annulaire (23) pour tenir lesdits moyens résistifs (R1, R2), formé dans l'épaisseur dudit fût cylindrique (22) et défini par une paroi interne cylindrique (24) placée autour du trou traversant axial (21) et par une paroi externe cylindrique (25) placée autour dudit fût, de sorte à ce que lesdits moyens résistifs (R1, R2) peuvent chauffer la mèche (102).

2. Élément chauffant selon la revendication 1, **caractérisé en ce que** ladite paroi externe cylindrique (25) qui délimite le siège (23) des moyens résistifs (R1, R2), présente des ouvertures (26) près des moyens résistifs, adaptées en vue de ne pas perdre de la chaleur en direction de l'extérieur, et de concentrer la chaleur en direction de la paroi interne (24) qui est en contact avec la mèche (102).

3. Élément chauffant selon la revendication 1, **caractérisé en ce que**, dans ladite paroi externe (25) du fût (22), dans lequel se trouve le siège des moyens résistifs, il existe deux entailles (27) adaptées en vue de permettre le passage des câbles électriques (17, 17') reliés aux moyens résistifs (R1, R2).

4. Élément chauffant selon l'une quelconque des revendications précédentes,
**caractérisé en ce que** ledit fût (22) soutenant les moyens résistifs (R1, R2) est formé axialement dans ladite demi-coque supérieure (20; 220) ou respectivement dans ladite demi-coque inférieure (40; 240), et dans ladite demi-coque inférieure (40; 240) ou respectivement dans ladite demi-coque supérieure (20; 220), il existe un fût correspondant (42) présentant une surface de butée (43; 261) adaptée en vue de buter contre lesdits moyens résistifs (R1, R2) placés dans ledit siège (23) du premier fût (22) afin de les maintenir fixes en position.

5. Élément chauffant selon l'une quelconque des revendications précédentes, **caractérisé en ce que** lesdits moyens résistifs constituent deux résistances électriques (R1, R2).

6. Élément chauffant selon la revendication 5, **caractérisé en ce que** lesdits moyens d'alimentation constituent une fiche électrique (10) comprenant deux broches (12) reliées par des câbles électriques (17, 17') auxdites deux résistances électriques (R1, R2).

7. Élément chauffant selon la revendication 5 ou 6, **caractérisé en ce que** lesdites résistances sont recouvertes par une gaine d'isolation résistante à la chaleur (75) faisant office de protection contre une éventuelle détérioration de l'élément chauffant.

8. Élément chauffant selon la revendication 6 ou 7, **caractérisé en ce que** ladite fiche électrique (10) présente une bride circulaire (13) adaptée au connecteur de couplage rotatif respectif (30, 50; 232, 252) dudit élément chauffant, de sorte qu'elle puisse être tournée afin de s'adapter à l'orientation de la douille d'alimentation électrique dans laquelle elle doit être insérée.

9. Élément chauffant selon l'une quelconque des revendications précédentes, **caractérisé en ce qu'**il comprend des dispositifs de régulation adaptés en vue de déplacer le récipient (101) et/ou la mèche (102) de sorte à commander la quantité de solution diffusée pendant l'unité de temps.

10. Élément chauffant selon l'une quelconque des revendications précédentes, **caractérisé en ce que** lesdites demi-coques inférieure (40; 240) et supérieure (20; 220) sont formées à l'aide d'un moulage par injection de matières plastiques.

11. Élément chauffant selon la revendication 10, **caractérisé en ce que** lesdites demi-coques inférieure (40; 240) et supérieure (20; 220) sont constituées de matière plastique ayant une résistance adéquate à la chaleur, telle que le polypropylène (PP).

12. Élément chauffant selon la revendication 10, **caractérisé en ce que**, au moins les parties desdites demi-coques inférieure (40; 240) et supérieure (20; 220) proches desdits éléments résistifs, sont constituées d'une matière plastique ayant de bonnes caractéristiques de résistance à la chaleur, telle que PBT (poly-téréphtalate de butylène) ou PPS (poly-sulfure de phényle).

13. Élément chauffant selon l'une quelconque des revendications précédentes, **caractérisé en ce que** lesdites demi-coques inférieure (40; 240) et supérieure (20; 220) sont couplées ensemble en permanence par une soudure, telle qu'un thermo-scellage ou une soudure par ultrasons.

14. Élément chauffant selon l'une quelconque des revendications 1 à 12, **caractérisé en ce que** lesdites demi-coques inférieure (40; 240) et supérieure (20; 220) sont couplées ensemble en permanence au moyen de couplages fixes ou à encliquetage.

15. Dispositif diffuseur (100), comprenant un récipient (101) contenant une solution à diffuser, et une mèche (102) plongée au moins partiellement dans ladite solution à diffuser, contenue dans le récipient (101), **caractérisé en ce qu'**il comprend un élément chauffant (1; 200) selon l'une quelconque des revendications précédentes.
